Europäisches Patentamt

European Patent Office

Office européen des brevets

(11) Veröffentlichungsnummer: **0 011 225**
**B1**

(12) **EUROPÄISCHE PATENTSCHRIFT**

(19)

(45) Veröffentlichungstag der Patentschrift:
**12.05.82**

(21) Anmeldenummer: **79104353.2**

(22) Anmeldetag: **07.11.79**

(51) Int. Cl.³: **B 01 J 23/88,** B 01 J 27/18,
C 07 C 5/48, C 07 C 15/46

(54) **Trägerkatalysator, Verfahren zu seiner Herstellung und seine Verwendung zur Herstellung von Styrol.**

(30) Priorität: **16.11.78 DE 2849715**

(43) Veröffentlichungstag der Anmeldung:
**28.05.80 Patentblatt 80/11**

(45) Bekanntmachung des Hinweises auf die Patenterteilung:
**12.05.82 Patentblatt 82/19**

(84) Benannte Vertragsstaaten:
**BE DE FR GB IT NL**

(56) Entgegenhaltungen:
**keine**

(73) Patentinhaber: **HOECHST AKTIENGESELLSCHAFT,
Postfach 80 03 20, D-6230 Frankfurt/Main 80 (DE)**

(72) Erfinder: **Erpenbach, Heinz, Dr., Oberbuschweg 22,
D-5000 Köln 50 (DE)**
Erfinder: **Gehrmann, Klaus, Dr.,
Geschwister-Scholl-Strasse 32, D-5042 Erftstadt (DE)**
Erfinder: **Joest, Herbert, Heddinghovener Strasse 3,
D-5042 Erftstadt (DE)**

### Trägerkatalysator, Verfahren zu seiner Herstellung und seine Verwendung
### zur Herstellung von Styrol

Die Erfindung betrifft einen Trägerkatalysator, ein Verfahren zu einer Herstellung sowie seine Verwendung zur Herstellung von Styrol durch oxidative Dehydrierung von Ethylbenzol mit molekularem Sauerstoff in der Gasphase.

Es sind bereits Trägerkatalysatoren für die genannte Umsetzung bekanntgeworden. So beschreibt die GB-PS 1 148 108 die oxidative Dehydrierung von Ethylbenzol mit Luft in Gegenwart von Wasserdampf bei 350° bis 600°C unter Einsatz eines Fließbettkatalysators, bestehend aus Chromoxid und Alkalimetalloxid auf einem Trägermaterial. Gemäß US-PS 3 917 732 wird die oxidative Dehydrierung in Gegenwart von Magnesium-Nickel-Pyrophosphat als Katalysator und von Helium, Stickstoff oder Wasserdampf als inertem Verdünnungsmittel durchgeführt. Die US-PS 3 923 916 beschreibt die oxidative Dehydrierung in Gegenwart von Nickelpyrophosphat als Katalysator und von Helium als inertem Verdünnungsmittel. Die US-PS 3 935 126 betrifft die genannte oxidative Dehydrierung in Gegenwart eines Erdalkali-Nickel-Phosphats als Katalysator und von Stickstoff oder Helium als inertem Verdünnungsmittel. Die US-PS 3 957 897 beschreibt dieselbe Umsetzung mit einem Erdalkalipyrophosphat-Katalysator und Helium als inertem Verdünnungsmittel.

Die meisten der bekannten Katalysatorsysteme sind für die großtechnische Herstellung von Styrol wenig geeignet, da sie den Einsatz von inerten Verdünnungsmitteln wie Helium oder Stickstoff erfordern, die entweder eine energieaufwendige Kreislaufführung nach sich ziehen oder infolge Tiefkühlung bzw. Wäsche die Gewinnung des gebildeten Styrols aus den Inertgasen sehr erschweren. Vor allem aber erlauben die bekannten Katalysatoren bei der erforderlichen hohen Selektivität nur eine unzureichende Produktivität von höchstens 135 g Styrol je Liter Katalysator und Stunde, die überdies nur in kleinsten Apparateeinheiten aufgrund gaschromatographischer Analyse des Gasstroms festgestellt werden kann, während bei der technischen Gewinnung von Styrol gemäß GB-PS 1 148 108 eine Produktivität von lediglich 75 g Styrol/l · h errechenbar ist.

Aufgabe der vorliegenden Erfindung ist die Vermeidung dieser Nachteile durch Herstellung geeigneter oxidischer Trägerkatalysatoren, die eine technisch durchführbare und zugleich wirtschaftliche oxydative Dehydrierung von Ethylbenzol zu Styrol in der Gasphase ermöglichen.

Die Erfindung betrifft nun einen Trägerkatalysator aus Oxiden des Kobalts und Phosphors sowie mindestens einem der Oxide des Molybdäns und des Kaliums im Atomverhältnis $Co_1P_{1-2}Mo_{0-0,05}K_{0-0,5}$ auf einem porösen Trägermaterial.

Der Trägerkatalysator der Erfindung kann weiterhin bevorzugt und wahlweise dadurch gekennzeichnet sein, daß

a) das Trägermaterial eine BET-Oberfläche von 0,1 bis 500, vorzugsweise 2 bis 200 m²/g, besitzt;
b) das Trägermaterial eine Korngröße von 0,01 bis 6 mm, vorzugsweise von 0,01 bis 0,2 mm für den Einsatz im Fließbett oder von 3 — 6 mm für den Einsatz im Festbett, aufweist;
c) er 2 bis 30 Gew.-% der Oxide des Kobalts und Phosphors sowie des Molybdäns und/oder Kaliums enthält und
d) er Kieselsäure oder Aluminiumoxid als poröses Trägermaterial enthält.

Die Erfindung betrifft ebenso ein Verfahren zur Herstellung des Trägerkatalysators, welches dadurch gekennzeichnet ist, daß man

a) ein trockenes, poröses Trägermaterial bis zum Erreichen von 40 bis 80% des vorher ermittelten Sättigungswertes mit Wasser tränkt;
b) das vorgetränkte Trägermaterial entweder mindestens einmal mit einer höchstens zur vollständigen Sättigung ausreichenden Menge einer wäßrigen Lösung von wasserlöslichen Verbindungen des Kobalts und Phosphors sowie des Molybdäns und/oder Kaliums oder nacheinander, jedoch mit zwischenzeitlichen Trocknungsintervallen gemäß c), mit einer höchstens zur vollständigen Sättigung ausreichenden Menge einer wäßrigen Lösung einer wasserlöslichen Verbindung von jeweils nur einem der genannten Elemente imprägniert;
c) das Trägermaterial nach jeder Imprägnierung 2 bis 20 Stunden bei 350 bis 500 K (77 bis 227°C) trocknet und
d) abschließend 0,5 bis 4 Stunden bei 550 bis 1000 K (227 bis 727°C), vorzugsweise bei 600 bis 900 K (327 bis 627°C) im Luftstrom sintert.

Das vorgetränkte Trägermaterial wird dabei vorzugsweise bei Temperaturen von 290 bis 375 K (17 bis 102°C) imprägniert.

Schließlich betrifft die Erfindung auch die Verwendung des Trägerkatalysators zur Herstellung von Styrol durch oxidative Dehydrierung von Ethylbenzol mit molekularem Sauerstoff in der Gasphase sowie das auf diese Weise hergestellte Styrol.

Die Erfindung ermöglicht die genannte Umsetzung mit gleichzeitig hoher Selektivität und Produktivität. Die folgenden Begriffe seien hiermit definiert:

$$\text{Umsatz (\%)} = \frac{\text{Mol eingesetztes Ethylbenzol/h} - \text{Mol aus Reaktionsprodukt rückgewonnenes Ethylbenzol/h}}{\text{Mol eingesetztes Ethylbenzol/h}} \cdot 100$$

$$\text{Ausbeute (\%)} = \frac{\text{Mol gewonnenes Styrol/h}}{\text{Mol eingesetztes Ethylbenzol/h}} \cdot 100$$

$$\text{Selektivität (\%)} = \frac{\text{Ausbeute}}{\text{Umsatz}} \cdot 100$$

$$\text{Produktivität} = \frac{\text{g gewonnenes Styrol}}{\text{l Katalysator} \cdot \text{h}}$$

Bei der Herstellung des erfindungsgemäßen Katalysators setzt man als wasserlösliche Verbindung zweckmäßig wasserlösliche Metallsalze und Phosphorsäure ein. Kobalt und Kalium werden bevorzugt als Nitrate, aber auch als Chloride, Carbonate und organische Säuresalze (Formiate, Acetate, Citrate) eingesetzt. Molybdän wird zweckmäßig in Form von Ammoniummolybdat, besonders Ammoniumheptamolybdat, oder zusammen mit Kalium als Kaliummolybdat in Lösung gebracht. Phosphor und Kalium können auch gemeinsam in Form eines Kaliumphosphats eingesetzt werden.

Bei der Herstellung des erfindungsgemäßen Katalysators wird die Tränkung des vorzugsweise kugelförmigen $SiO_2$- oder $Al_2O_3$-Trägermaterials mit Wasser bis zum Erreichen von $40-80\%$ seines Aufnahmevermögens vorgenommen, damit die anschließend gemeinsam oder einzeln nach und nach aufgetragenen gelösten aktiven Verbindungen überwiegend an der Oberfläche des Trägers angereichert werden.

Die oxidative Dehydrierung von Ethylbenzol zu Styrol kann in Gegenwart des Trägerkatalysators der Erfindung wie folgt durchgeführt werden:

Ethylbenzol und Sauerstoff werden im Molverhältnis von 1 zu $0,1-1$ in Gegenwart von $1-5$ Mol Wasserdampf und $0-4$ Mol Inertgas wie $N_2$ oder $CO_2$ je Mol Ethylbenzol bei Temperaturen von 550 bis 1000 K und $1-5$ bar Druck durch oder über den Trägerkatalysator der Erfindung geleitet. Hierbei kann im Fest-, Fließ- oder Wirbelbett gearbeitet werden.

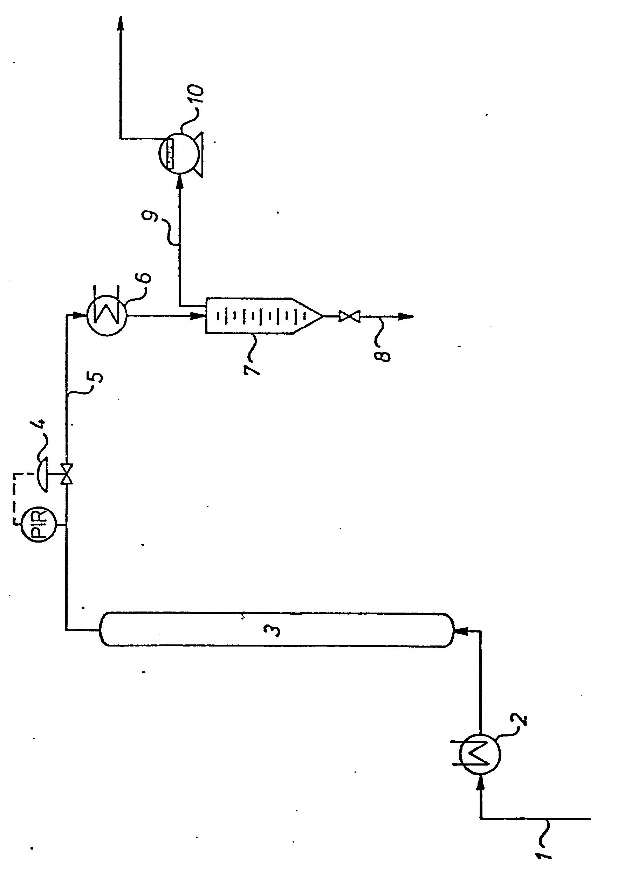

Ein mögliches Fließschema zur Durchführung des Verfahrens ist in der Zeichnung dargestellt und im folgenden beschrieben:

Ethylbenzol, reiner Sauerstoff oder Luft, Wasserdampf und ggf. Stickstoff oder Kohlendioxid werden über Leitung (1) und den Wärmetauscher (2) dem ummantelten Reaktor (3) zugeführt. Im Wärmetauscher (2) wird die Ausgangsmischung auf $400-550$ K ($127-277°$C) vorgeheizt. Die gewünschte Reaktionstemperatur von $550-1000$ K ($277-727°$C), vorzugsweise $600-900$ K ($327-627°$C), wird mittels eines im Mantelraum befindlichen elektrisch beheizten Sand- oder Salzbades eingestellt. Der Reaktionsdruck wird über ein automatisches Regelventil (4) auf 1 bis 5, vorzugsweise 1 bis 3 bar gehalten. Die Belastung des Katalysators mit gasförmiger Ausgangsmischung erfolgt mit einer Raumgeschwindigkeit von 100 bis 3000, vorzugsweise 500 bis 1500 $h^{-1}$ (Raumgeschwindigkeit = Nl Ausgangsmischung, dividiert durch Liter Katalysator im Reaktor $\cdot$ h). Hieraus ergibt sich eine Verweilzeit der Ausgangsmischung am Katalysator von 0,1 bis 30 s, vorzugsweise 0,5 bis 6 s. Die Reaktionsgase verlassen den Reaktor (3) über Leitung (5) und den Kondensator (6) und trennen sich im Abscheider (7) in ein flüssiges Kondensat und Abgas. Die kondensierten Reaktionsprodukte werden über die Leitung (9) abgezogen, gewichtsmäßig erfaßt und gaschromatographisch analysiert. Das Abgas verläßt das System über die Leitung (9). Die Abgasmenge wird durch die Gasuhr (10) erfaßt und seine Zusammensetzung gaschromatographisch ermittelt. Das erfindungsgemäße Verfahren wird durch die nachstehenden Beispiele erläutert.

## Beispiel 1

In einem Rotationsverdampfer werden 580 g $SiO_2$-Kugeln (Durchmesser: 5 mm; BET-Oberfläche 40 m²/g) mit 500 g Wasser entsprechend 71% des Sättigungswertes vorgetränkt und anschließend mit einer Lösung aus 200 g Wasser, 55,1 g Co $(NO_3)_2 \cdot 6 H_2O$, 32,86 g $H_3PO_4$ (85%ig) und 2,87 g $KNO_3$ bei einer Temperatur von 370 K (97°C) kontinuierlich belegt. Nach 16stündiger Trocknung bei 400 K (127°C) erfolgt im Luftstrom eine Sinterung während 1 h bei 823 K (550°C). Der Katalysator enthält 6,2 Gew.-% katalytisch aktive Elemente in Form ihrer Oxide in der Zusammensetzung $Co_1P_{1,5}K_{0,15}O_{5,325}$.

## Beispiel 2

Gemäß Beispiel 1 werden 600 g $SiO_2$-Kugeln (Durchmesser: 5 mm; BET-Oberfläche: 45 m²/g) mit 400 g Wasser entsprechend 45% des Sättigungswertes vorgetränkt und mit einer Lösung von 490 g Wasser, 200,9 g Co $(NO_3)_2 \cdot 6 H_2O$, 119,4 g $H_3PO_4$ (85%ig) und 1,21 g $(NH_4)_6Mo_7O_{24} \cdot 4 H_2O$ belegt. Nach Trocknung (16 h/127°C) und Sinterung (1 h/550°C) erhält man einen Katalysator mit 18 Gew.-% katalytisch aktiver Masse der Zusammensetzung $Co_1P_{1,5}Mo_{0,01}O_{5,28}$.

## Beispiel 3

In einem Plastikbeutel werden 400 g $SiO_2$ mit einer Korngröße von 0,01 – 0,2 mm und einer BET-Oberfläche von 180 m²/g mit 200 g Wasser entsprechend 50% des Sättigungswertes vorgetränkt. Anschließend wird eine Lösung von 600 g Wasser, 65,4 g $Co(NO_3)_2 \cdot 6 H_2O$, 38,8 g $H_3PO_4$ (85%ig), 3,42 g $KNO_3$ und 0,39 g $(NH_4)_6Mo_7O_{24} \cdot 4 H_2O$ in drei Auftragungen bei einer Temperatur von 295 K (22°C) mit dem Träger verknetet. Nach jeder Auftragung wird die Katalysatormasse bei 400 K (127°C) über 2 h getrocknet. Abschließend erfolgt die Sinterung (1 h/550°C) im Luftstrom. Der Katalysator enthält 10 Gew.-% katalytisch aktive Masse der Zusammensetzung $Co_1P_{1,5}Mo_{0,01}K_{0,15}O_{5,355}$.

## Beispiel 4

Gemäß Beispiel 3 werden 630 g $Al_2O_3$ mit einer Korngröße von 0,01 – 0,2 mm und einer BET-Oberfläche von 20 m²/g mit 250 g Wasser entsprechend 56% des Sättigungswertes vorgetränkt und anschließend mit einer Lösung von 200 g Wasser, 164,8 g $Co(NO_3)_2 \cdot 6 H_2O$, 97,8 g $H_3PO_4$ (85%ig) und 8,54 g $KNO_3$ belegt. Trocknung und Sinterung entsprechen Beispiel 3. Der Katalysator enthält 15,3 Gew.-% katalytisch aktive Masse der Zusammensetzung $Co_1P_{1,5}K_{0,15}O_{5,325}$.

## Beispiel 5

512 g (1 l) des gemäß Beispiel 1 hergestellten Katalysators $Co_1P_{1,5}K_{0,15}O_{5,325}$ werden in einem Festbettreaktor (1,2 l Inhalt, 121 cm Füllhöhe) stündlich mit 1440 Nl eines Gemisches von 13 Vol.-% Ethylbenzol, 31 Vol.-% Luft und 56 Vol.-% Wasserdampf beschickt. Bei einem Reaktordruck von 2,5 bar, einer Reaktionstemperatur von 819 K (546°C) sowie einer Verweilzeit von 2,1 s werden 37% des Ethylbenzols umgesetzt. Die Ausbeute an Styrol beträgt 30,8% und die Selektivität 83,3%. Die Produktivität beläuft sich auf 270 g Styrol/l Katalysator · h.

## Beispiel 6

595 g (1 l) Katalysator $Co_1P_{1,5}Mo_{0,01}O_{5,28}$ gemäß Beispiel 2 werden im Festbettreaktor stündlich mit 1004 Nl eines Gemisches von 12,6 Vol.-% Ethylbenzol, 31,6 Vol.-% Luft und 55,8 Vol.-% Wasserdampf beschickt. Bei einem Reaktordruck von 1,1 bar, einer Reaktionstemperatur von 784 K (511°C) sowie einer Verweilzeit von 1,3 s werden 39,3% des Ethylbenzols umgesetzt. Bei einer Ausbeute von 33,7% ergeben sich 85,7% Styrol-Selektivität. Die Produktivität beträgt 199 g Styrol/l Katalysator · h.

## Beispiel 7

382 g (0,9 l) Katalysator $Co_1P_{1,5}Mo_{0,01}K_{0,15}O_{5,355}$ gemäß Beispiel 3 werden in einem Wirbelbettreaktor (2 l Reaktionsraum, 50 cm Füllhöhe) mit stündlich 853 Nl (Raumgeschwindigkeit 948 h⁻¹) eines Gemisches von 13,2 Vol.-% Ethylbenzol, 31,2 Vol.-% Luft und 55,6 Vol.-% Wasserdampf beschickt. Bei einem Reaktordruck von 1,1 bar, einer Reaktionstemperatur von 848 K (575°C) sowie 1,3 s Verweilzeit werden 40,3% des Ethylbenzols umgesetzt. Bei einer Ausbeute von 35,5% ergibt sich eine Styrol-Selektivität von 88,2%. Die Produktivität beträgt 205 g Styrol/l Katalysator · h.

## Beispiel 8

640 g (0,8 l) Katalysator $Co_1P_{1,5}K_{0,15}O_{5,325}$ gemäß Beispiel 4 werden im Wirbelbettreaktor stündlich mit 1040 Nl (Raumgeschwindigkeit 1300 h⁻¹) eines Gemisches von 12,6 Vol.-% Ethylbenzol, 30 Vol.-% Luft und 57,4 Vol.-% Wasserdampf beschickt. Bei einem Reaktordruck von 1,1 bar, einer Reaktionstemperatur von 764 K (491°C) sowie 1,2 s Verweilzeit werden 32,4% des Ethylbenzols umgesetzt. Styrolausbeute: 28%; Selektivität: 86,3%; Produktivität: 213 g Styrol/l Katalysator · h.

4

**Patentansprüche**

1. Trägerkatalysator aus Oxiden des Kobalts und Phosphors sowie mindestens einem der Oxide des Molybdäns und des Kaliums im Atomverhältnis $Co_1P_{1-2}Mo_{0-0,05}K_{0-0,5}$ auf einem porösen Trägermaterial.

2. Trägerkatalysator nach Anspruch 1, dadurch gekennzeichnet, daß das Trägermaterial eine BET-Oberfläche von 0,1 bis 500, vorzugsweise 2 bis 200 m²/g, besitzt.

3. Trägerkatalysator nach Anspruch 1 oder 2, dadurch gekennzeichnet, daß das Trägermaterial eine Korngröße von 0,01 bis 6 mm, vorzugsweise von 0,01 bis 0,2 mm für den Einsatz im Fließbett oder von 3 — 6 mm für den Einsatz im Festbett, aufweist.

4. Trägerkatalysator nach einem der Ansprüche 1 bis 3, dadurch gekennzeichnet, daß er 2 bis 30 Gew.-% der Oxide des Kobalts und Phosphors sowie des Molybdäns und/oder Kaliums enthält.

5. Trägerkatalysator nach einem der Ansprüche 1 bis 4, dadurch gekennzeichnet, daß er Kieselsäure oder Aluminiumoxid als poröses Trägermaterial enthält.

6. Verfahren zur Herstellung eines Trägerkatalysators gemäß Anspruch 1, dadurch gekennzeichnet, daß man

a) ein trockenes, poröses Trägermaterial bis zum Erreichen von 40 bis 80% des vorher ermittelten Sättigungswertes mit Wasser tränkt;

b) das vorgetränkte Trägermaterial entweder mindestens einmal mit einer höchstens zur vollständigen Sättigung ausreichenden Menge einer wäßrigen Lösung von wasserlöslichen Verbindungen des Kobalts und Phosphors sowie des Molybdäns und/oder Kaliums oder nacheinander, jedoch mit zwischenzeitlichen Trocknungsintervallen gemäß c), mit einer höchstens zur vollständigen Sättigung ausreichenden Menge einer wäßrigen Lösung einer wasserlöslichen Verbindung von jeweils nur einem der genannten Elemente imprägniert;

c) das Trägermaterial nach jeder Imprägnierung 2 bis 20 Stunden bei 350 bis 500 K (77 bis 227° C) trocknet und

d) abschließend 0,5 bis 4 Stunden bei 550 bis 1000 K (277 bis 727° C), vorzugsweise bei 600 bis 900 K (327 bis 627° C), im Luftstrom sintert.

7. Verfahren nach Anspruch 6, dadurch gekennzeichnet, daß man das vorgetränkte Trägermaterial gemäß b) bei Temperaturen von 290 bis 375 K (17 bis 102° C) imprägniert.

8. Verwendung des Trägerkatalysators gemäß Anspruch 1 zur Herstellung von Styrol durch oxidative Dehydrierung von Ethylbenzol mit molekularem Sauerstoff in der Gasphase.

9. Styrol, hergestellt durch oxidative Dehydrierung von Ethylbenzol mit molekularem Sauerstoff in der Gasphase in Gegenwart eines Trägerkatalysators gemäß Anspruch 1.


**Claims**

1. Carrier-supported catalyst comprising oxides of cobalt and phosphorus and at least one of the oxides of molybdenum and potassium in the atomic ratio of $Co_1P_{1-2}Mo_{0-0,05}K_{0-0,5}$ on a porous carrier material.

2. Carrier-supported catalyst as claimed in claim 1, characterized in that the carrier material has a BET-surface area of 0.1 to 500 m²/g, preferably 2 to 200 m²/g.

3. Carrier-supported catalyst as claimed in claim 1 or 2, characterized in that the carrier material consists of particles with a size of 0.01 to 6 mm, preferably 0.01 to 0.2 mm for use in a flow bed, or 3 to 6 mm for use in a fixed bed.

4. Carrier-supported catalyst as claimed in any of claims 1 to 3, characterized in that it contains 2 to 30 weight % of the oxides of cobalt and phosphorus as well as of molybdenum and/or potassium.

5. Carrier-supported catalyst as claimed in any of claims 1 to 4, characterized in that it contains silicic acid or aluminum oxide as the porous carrier material.

6. Process for making a carrier-supported catalyst as claimed in claim 1, characterized in that:

a) a dry porous carrier material is impregnated with water up to 40 to 80% of its predetermined saturation value;

b) the carrier material so treated is impregnated either at least once with an aqueous solution of water-soluble compounds of cobalt and phosphorus as well as of molybdenum and/or potassium, or repeatedly and successively, each impregnation being interrupted by an intermediary drying period as specified under c), with an aqueous solution of a water-soluble compound of merely one of the elements aforesaid, the aqueous solution being used in either case in a quantity which is at most necessary for complete saturation;

c) the carrier material is dried, after each impregnation, over a period of 2 to 20 hours at 350 to 500 K (77 to 227° C); and

d) the carrier material is finally sintered over a period of 0.5 to 4 hours at 550 to 1000 K (277 to 727°C), preferably 600 to 900 K (327 to 627°C) in a stream of air.

7. Process as claimed in claim 6, characterized in that the carrier material pre-impregnated as described under b) is further im...regnated at temperatures of 290 to 375 K (17 to 102°C).

8. Use of the carrier-supported catalyst as claimed in claim 1 for the production of styrene by subjecting ethylbenzene to oxidative dehydrogenation with molecular oxygen in gas phase.

9. Styrene prepared by subjecting ethylbenzene to oxidative dehydrogenation with molecular oxygen in gas phase, in the presence of a carrier-supported catalyst as claimed in claim 1.

## Revendications

1. Catalyseur sur support constitué par des oxydes de cobalt et de phosphore et au moins l'un des oxydes de molybdène et de potassium dans un rapport atomique de $Co_1P_{1-2}Mo_{0-0,05}K_{0-0,5}$ sur un support poreux.

2. Catalyseur sur support selon la revendication 1, caractérisé en ce que le support présente une surface BET de 0,1 à 500, de préférence 2 à 200 $m^2/g$.

3. Catalyseur sur support selon la revendication 1 ou 2, caractérisé en ce que le support consiste en particules d'une granulométrie de 0,01 à 6 mm, de préférence 0,01 à 0,2 mm, pour utilisation dans un lit fluide, ou de 3 à 6 mm pour utilisation dans un lit fixe.

4. Catalyseur sur support selon l'une des revendications 1 à 3, caractérisé en ce qu'il contient 2 à 30% en poids d'oxydes de cobalt et de phosphore ainsi que de molybdène et/ou de potassium.

5. Catalyseur sur support selon l'une des revendications 1 à 4, caractérisé en ce qu'il contient de l'acide silicique ou de l'oxyde d'aluminium comme support poreux.

6. Procédé de préparation d'un catalyseur sur support selon la revendication 1, caractérisé en ce que:

a) on imprègne un support poreux sec par l'eau à un taux de 40—80% de sa valeur de saturation déterminée au préalable;

b) on imprègne le support ainsi traité soit au moins une fois par une solution aqueuse de composés hydrosolubles de cobalt et de phosphore ainsi que de molybdène et/ou de potassium, soit à plusieurs reprises, chaque imprégnation étant alors suivie d'une période de séchage intermédiaire selon c), par une solution aqueuse ne contenant qu'un composé hydrosoluble de l'un des éléments spécifiés, la solution aqueuse étant chaque fois utilisée en quantité maximale nécessaire pour saturation complète;

c) après chaque imprégnation, on sèche pendant 2 à 20 heures à 350—500 K (77 à 227°C) et

d) on fritte enfin pendant 0,5 à 4 heures à 550—1000 K (277 à 727°C), de préférence 600—900 K (327 à 627°C) dans un courant d'air.

7. Procédé selon la revendication 6, caractérisé en ce que l'on imprègne le support traité selon b) à des températures de 290 à 375 K (17 à 102°C).

8. Utilisation du catalyseur sur support selon la revendication 1 pour la préparation du styrène par déshydrogénation oxydante de l'éthylbenzène à l'aide d'oxygène moléculaire en phase gazeuse.

9. Styrène, préparé par déshydrogénation oxydante de l'éthylbenzène à l'aide d'oxygène moléculaire en phase gazeuse en présence d'un catalyseur sur support selon la revendication 1.